# FASCICULE DE BREVET EUROPEEN

(11) **EP 0 379 455 B1**
(45) Date de publication et mention de la délivrance du brevet: **09.02.1994**
(21) Numéro de dépôt: 90420030.0
(22) Date de dépôt: 19.01.1990
(51) Int. Cl.: A61L 2/18, A61C 19/00

(54) **Dispositif de nettoyage et de désinfection d'instruments médicaux et chirurgicaux**
Reinigungs- und Desinfektionsvorrichtung für medizinische und chirurgische Geräte
Device for cleaning and disinfecting medical and surgical instruments

(30) Priorité: 20.01.1989 FR 8901306
(43) Date de publication de la demande: 25.07.1990
(73) Titulaire: Bene, Pierre-Yves, F-69006 Lyon (FR)
(72) Inventeur: Bene, Pierre-Yves, F-69006 Lyon (FR)
(74) Mandataire: Maureau, Philippe

(56) Documents cités:
- EP-A- 0 056 791
- EP-A- 0 287 481

## Description

La présente invention a pour objet un dispositif de nettoyage et de désinfection d'instruments médicaux et chirurgicaux.

Les praticiens, qu'ils s'agissent de chirurgiens, de chirurgiens dentistes ou de médecins utilisent des instruments qu'il n'est pas toujours possible de stériliser ou de désinfecter parfaitement entre deux interventions successives. Tel est notamment le cas des instruments qui sont solidaires de la console de soin et dont le nettoyage complet ne peut être réalisé qu'après un démontage long, qui ne peut être effectué entre deux interventions successives. Ces instruments sont par exemple des pièces à main, contre-angle, appareils à ultra-sons et vaporisateurs à trois voies utilisés en permanence par les chirurgiens dentistes.

Or, ces instruments pouvant subir des souillures importantes à partir de microbes ou virus contenus à l'intérieur de dents gangrénées, dans la plaque dentaire, dans la salive ou dans le sang, il est important, dans la mesure du possible, de procéder au retrait de ces souillures pour éviter les risques de contamination d'un patient à un autre, ou d'un patient vis-à-vis du personnel médical.

De telles contaminations ont des conséquences particulièrement graves dans le cas de maladies difficiles à traiter, telles que l'hépatite B, ou pour lesquelles on ne dispose pas encore de traitements, telles que le SIDA.

Pour remédier à ces inconvénients, il a été imaginé de réaliser un dispositif comprenant un corps tubulaire dont une extrémité permettant l'introduction de l'instrument à nettoyer est ouverte, et dont l'autre extrémité est équipée d'un réservoir de recueil de liquide. La paroi intérieure du corps tubulaire est équipée de buses orientées sensiblement radialement, diffusant, après introduction d'un instrument dans la cavité du corps, un produit de désinfection nébulisé. Ce produit réalise d'une part un nettoyage mécanique de l'instrument, et d'autre part assure une désinfection de celui-ci, les souillures et le liquide nébulisé étant dirigées vers le réservoir de recueil de liquide, ce mouvement étant facilité par la dépression créée par un aspirateur, et la condensation du nuage liquide étant facilité par un système de chicanes.

Toutefois, les appareils connus de ce type ne donnent pas entière satisfaction du fait des difficultés qu'il y a à réaliser une alimentation correcte des injecteurs, notamment en liquide de désinfection, surtout si le liquide n'est pas amené sous pression, mais est simplement aspiré par le passage de l'air comprimé au niveau des injecteurs. En effet, il convient de réaliser un système dans lequel l'amenée du liquide soit la plus équilibrée possible, et dans lequel une bonne étanchéité soit réalisée au niveau des pièces dans lesquels sont ménagés les canaux de distribution de fluide.

La présente invention vise à remédier aux inconvénients des appareils existants en fournissant un dispositif dans lequel les moyens d'alimentation en air et en liquide des différents injecteurs, sont simples tout en possèdant toutes les qualités requises notamment au niveau de l'équilibrage des différents injecteurs.

A cet effet, le dispositif de nettoyage et de désinfection d'instruments médicaux et chirurgicaux qu'elle concerne, du type comprenant un corps tubulaire dont une extrémité est ouverte et dont l'autre extrémité débouche dans un réservoir de recueil de liquides usés, et dont la paroi interne est équipée de buses de diffusion d'un produit de désinfection nébulisé, chaque buse étant associée à un injecteur auquel sont amenés le liquide de désinfection et de l'air sous pression, est caractérisé en ce que les moyens d'alimentation en air des injecteurs comprennent un conduit annulaire et circulaire s'étendant sur 360°, disposé autour du corps tubulaire, dans un plan transversal à l'axe de celui-ci, alimentant directement les différents injecteurs, et en ce que les moyens d'alimentation en liquide de désinfection comprennent un premier conduit circulaire s'étendant sur 180°, disposé autour du corps tubulaire, dans un plan perpendiculaire à l'axe de celui-ci, alimenté en son milieu par le liquide de désinfection, et dans les deux extrémités duquel débouchent deux conduits axiaux dont l'autre extrémité de chacun débouche au milieu d'un conduit circulaire s'étendant sur 90° dans un plan transversal à l'axe du corps tubulaire, les deux extrémités de chacun de ces derniers conduits communiquant avec un injecteur, ou avec un conduit dont les extrémités alimentent chacune un injecteur, selon le nombre d'injecteurs que comprend le dispositif.

La subdivision du conduit d'amenée de liquide en deux conduits qui se subdivisent chacun en deux conduits d'alimentation des injecteurs permet d'égaliser les distances parcourues par le liquide entre la source et chaque injecteur, de telle sorte que la charge est la même au niveau de tous les injecteurs. Si cette caractéristique est intéressante lorsque le liquide est amené sous pression, elle est encore plus importante lorsque le liquide est amené par simple gravité et aspiré par l'air sous pression.

Dans le cas où le dispositif comprend quatre injecteurs, les moyens d'alimentation en liquide de désinfection comprennent un premier conduit circulaire s'étendant sur 180°, disposé autour du corps tubulaire, dans un plan perpendiculaire à l'axe de celui-ci, alimenté en son milieu par le liquide de désinfection, et dans les deux extrémités duquel débouchent deux conduits axiaux dont l'autre extrémité de chacun débouche au milieu d'un conduit circulaire s'étendant sur 90° dans un plan transversal à l'axe du corps tubulaire, les deux extrémités de chacun de ces derniers conduits communiquant avec un injecteur.

Selon une forme d'exécution de ce dispositif, les moyens d'alimentation en air et en liquide de désinfection sont constitués par trois couronnes juxtaposées et solidarisées deux à deux, dans les faces en regard desquelles sont ménagées des rainures destinées à assurer la formation de canaux circulaires, la première couronne comprenant deux perçages axiaux pour le raccordement à des conduits d'amenée de liquide et d'air, les première et deuxième couronnes délimitant un premier canal s'étendant sur un angle de 180°, pour permettre le passage de liquide, la seconde couronne comportant trois perçages axiaux dont un pour le passage direct d'air et deux pour le passage de liquide, les deuxième et troisième couronnes délimitant d'une part sur 360° le canal circulaire de distribution d'air aux injecteurs, et d'autre part, sur 90° chacun deux canaux de distribution de liquide.

Selon une caractéristique de l'invention, les trois couronnes sont réalisées en matière synthétique et leurs faces en regard comportent des moyens d'assemblage constitués par une pluralité de bourrelets circulaires en vis-à-vis dont chacun est bordée par deux gorges, chaque rainure destinée à la formation d'un canal étant disposée entre deux bourrelets adjacents et de diamètre différent. Cette structure permet de réaliser un assemblage des différentes couronnes par la technique de soudure au miroir. Cette caractéristique est très intéressante car assurant un assemblage simple et rapide avec une excellente étanchéité, sans avoir à mettre en oeuvre ni joint d'étanchéité, ni joint de serrage.

De toute façon, l'invention sera bien comprise à l'aide de la description qui suit, en référence au dessin schématique annexé représentant à titre d'exemple non limitatif une forme d'exécution de ce dispositif :
Figure 1 est une vue en coupe longitudinale de la partie nettoyable proprement dite ;
Figure 2 est une vue en coupe transversale selon la ligne II-II de figure 1 ;
Figure 3 est une vue du schéma de distribution de l'air et du liquide de désinfection au niveau des injecteurs ;
Figure 4 est une vue en perspective éclatée des trois couronnes que comporte le dispositif ;
Figure 5 est une vue en coupe transversale de l'une de ces trois couronnes selon la ligne V-V de figure 4 ;
Figure 6 est une vue en coupe du détail de l'assemblage de deux couronnes.

Le dispositif selon l'invention comprend un corps tubulaire 2 dont l'extrémité avant élargie 3 permet l'introduction d'un instrument à nettoyer et dont l'extrémité arrière 4 permet le montage d'un réservoir de recueil de liquide. Dans la paroi intérieure du corps tubulaire débouchent quatre buses 5, décalées de 90° les unes par rapport aux autres, chaque buse étant alimentée par un injecteur 6 avec un mélange de liquide de désinfection et d'air afin de former un brouillard, l'air étant amené dans chaque injecteur à un niveau situé à l'extérieur du point d'amenée du liquide. Les moyens d'amenée du liquide et de l'air aux injecteurs 6 sont constitués par trois couronnes 7,8,9 disposées à l'extérieur du corps tubulaire, et solidarisées deux à deux les unes aux autres. La première couronne 7 comporte deux perçages en regard desquels sont ménagés deux embouts de raccordement 10 et 12 à des conduits d'amenée, respectivement d'air et de liquide de désinfection. En regard de l'embout et du perçage correspondant, la couronne 8 présente un perçage axial 13 amenant l'air dans un canal annulaire et circulaire 14 ménagé dans la couronne 9 s'étendant sur 360°. Le canal 14 alimente les quatre injecteurs 6 en air.

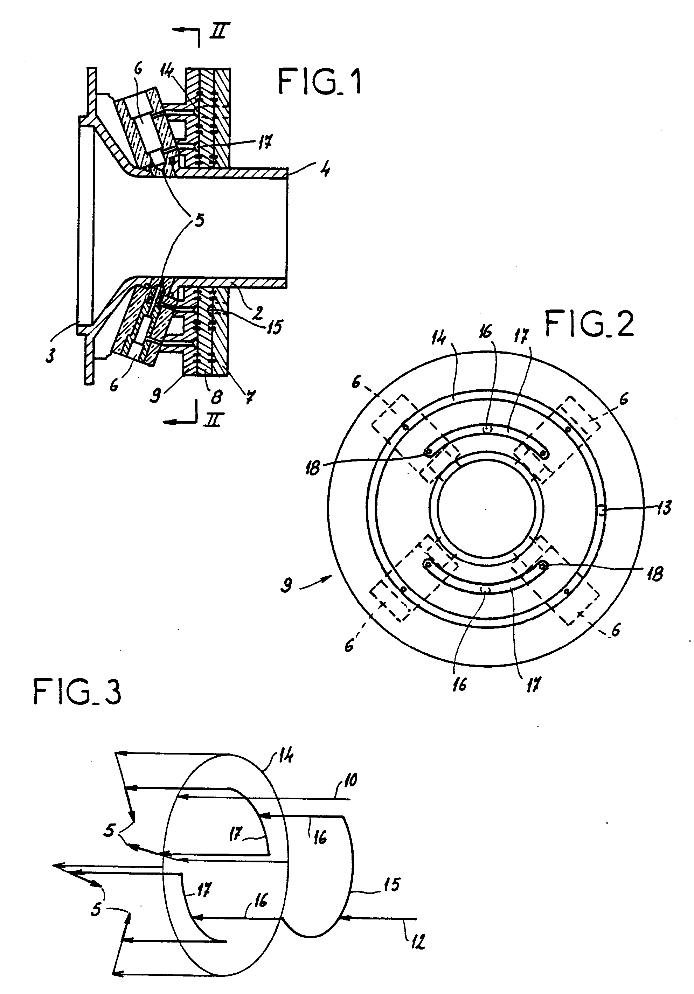

Pour sa part l'embout 12 alimente par le perçage ménagé dans la couronne 7, un canal 15 ménagé dans la couronne 8. Ce canal 15 s'étend sur 180°, à savoir 90° de part et d'autre de la position de l'embout 12. Aux extrémités du canal 15 sont ménagés des perçages 16 traversant la couronne 8 et alimentant en leur milieu deux canaux 17 qui s'étendant chacun sur 90° sont ménagés dans la couronne 9. A chaque extrémité d'un canal 17 est ménagé un perçage 18 assurant l'alimentation en liquide de désinfection d'un injecteur 6. Les circuits d'alimentation des injecteurs en air et en liquide sont schématisés à la figure 3. Il ressort de la description qui précède que l'alimentation en liquide des quatre injecteurs est effectuée de façon parfaitement équilibrée, de telle sorte que la nébulisation est la même au niveau de tous les injecteurs.

Afin d'assurer une parfaite étanchéité entre les différentes couronnes, sans nécessiter de joints ni de moyens de serrage, il est procédé à un assemblage de ces couronnes par une technique de soudure au miroir.

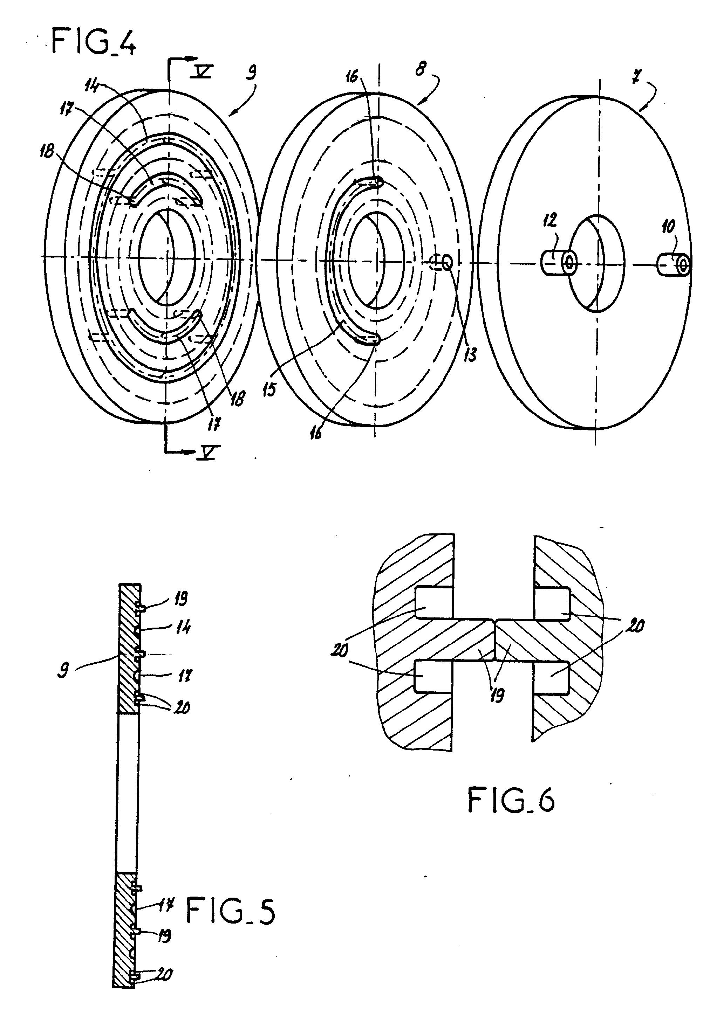

A cet effet, chaque couronne comporte, comme montré plus spécialement aux figures 5 et 6, trois lignes circulaires d'assemblage entre lesquelles sont disposés les canaux 14 et 17, pour ce qui concerne l'assemblage des couronnes 8 et 9. Chaque ligne d'assemblage résulte sur chacune des faces en regard de deux couronnes de la présence d'un bourrelet 19 de matière, de part et d'autre duquel sont ménagées deux gorges 20. Après amenée des couronnes à une température à laquelle les bourrelets sont dans un état malléable, les couronnes sont appliquées les unes contre les autres avec exercice d'une pression, mouvement au cours duquel est réalisée une solidarisation entre les bourrelets avec fluage de la matière résiduelle dans les gorges 20.

Comme il ressort de ce qui précède, l'invention apporte une grande amélioration à la technique existante en fournissant un dispositif de nettoyage et de désinfection d'instruments médicaux et chirurgicaux de conception simple, dans lequel l'amenée du liquide et de l'air est effectuée de façon parfaitement équilibrée.

Comme il va de soi, l'invention ne se limite pas à la seule forme d'exécution de ce dispositif décrite ci-dessus à titre d'exemple ; elle en embrasse, au contraire, toutes les variantes de réalisation. C'est ainsi notamment que le nombre d'injecteurs pourrait être différent, ou encore que le corps des injecteurs pourrait être perpendiculaire à l'axe du corps du dispositif, l'orifice de sortie des injecteurs étant alors incliné, sans que l'on sorte pour autant du cadre de l'invention.

## Revendications

1. Dispositif de nettoyage et de désinfection d'instruments médicaux et chirurgicaux, du type comprenant un corps tubulaire (2) dont une extrémité est ouverte et dont l'autre extrémité débouche dans un réservoir de recueil de liquides usés, et dont la paroi interne est équipée de buses (5) de diffusion d'un produit de désinfection nébulisé, chaque buse étant associée à un injecteur (6) auquel sont amenés le liquide de désinfection et de l'air sous pression, caractérisé en ce que les moyens d'alimentation en air des injecteurs (6) comprennent un conduit (14) annulaire et circulaire s'étendant sur 360°, disposé autour du corps tubulaire, dans un plan transversal à l'axe de celui-ci, alimentant directement les différents injecteurs (6), et en ce que les moyens d'alimentation en liquide de désinfection comprennent un premier conduit circulaire (15) s'étendant sur 180°, disposé autour du corps tubulaire, dans un plan perpendiculaire à l'axe de celui-ci, alimenté en son milieu par le liquide de désinfection, et dans les deux extrémités duquel débouchent deux conduits axiaux dont l'autre extrémité de chacun débouche au milieu d'un conduit circulaire (17) s'étendant sur 90°, dans un plan transversal à l'axe du corps tubulaire, les deux extrémités de chacun de ces derniers conduits communiquant avec un injecteur (6), ou avec un conduit dont les extrémités alimentent chacune un injecteur, selon le nombre d'injecteurs que comprend le dispositif.

2. Dispositif selon la revendication 1, caractérisé en ce que les moyens d'alimentation en liquide de désinfection comprennent un premier conduit circulaire s'étendant sur 180°, disposé autour du corps tubulaire, dans un plan perpendiculaire à l'axe de celui-ci, alimenté en son milieu par le liquide de désinfection, et dans les deux extrémités duquel débouchent deux conduits axiaux dont l'autre extrémité de chacun débouche au milieu d'un conduit circulaire s'étendant sur 90° dans un plan transversal à l'axe du corps tubulaire, les deux extrémités de chacun de ces derniers conduits communiquant avec un injecteur.

3. Dispositif selon la revendication 2, caractérisé en ce que les moyens d'alimentation en air et en liquide de désinfection sont constitués par trois couronnes (7,8,9) juxtaposées et solidarisées deux à deux, dans les faces en regard desquelles sont ménagées des rainures destinées à assurer la formation de canaux circulaires, la première couronne (7) comprenant deux perçages axiaux pour le raccordement à des conduits d'amenée de liquide et d'air, les première et deuxième couronnes (7,8) délimitant un premier canal (15) s'étendant sur un angle de 180°, pour permettre le passage de liquide, la seconde couronne (8) comportant trois perçages axiaux dont un (13) pour le passage direct d'air et deux (16) pour le passage de liquide, les deuxième et troisième couronnes (8,9) délimitant d'une part sur 360° le canal circulaire de distribution d'air (14) aux injecteurs (6), et d'autre part sur 90° chacun deux canaux (17) de distribution de liquide.

4. Dispositif selon la revendication 3, caractérisé en ce que les trois couronnes (7,8,9) sont réalisées en matière synthétique et leurs faces en regard comportent des moyens d'assemblage constitués par une pluralité de bourrelets circulaires (19) en vis-à-vis dont chacun est bordé par deux gorges (20), chaque rainure destinée à la formation d'un canal étant disposée entre deux bourrelets adjacents et de diamètre différent.

## Patentansprüche

1. Vorrichtung zur Reinigung und Desinfektion medizinischer und chirurgischer Instrumente, des Typs mit einem rohrförmigen Körper (2) dessen eines Ende offen ist und dessen anderes Ende in einen Sammelbehälter für gebrauchte Flüssigkeiten mündet und dessen Innenwandung mit Zerstreuungsdüsen für zerstäubtes Desinfektionsmittel ausgerüstet ist, wobei jede Düse einem Injektor (6) zugeordnet ist, welchem die Desinfektionsflüssigkeit und Druckluft zugeführt werden, dadurch gekennzeichnet, daß die Luft-Zufuhrmittel der Injektoren (6) eine ring- und kreisförmige, sich über 360° erstreckende Leitung (14) umfassen, welche um den rohrförmigen Körper herum in einer zu dessen Achse transversalen Ebene angeordnet ist und die verschiedenen Injektoren (6) direkt speist, und daß die Desinfektionsflüssigkeits-Zufuhrmittel eine erste kreisförmige, sich über 180° erstreckende Leitung (15) umfassen, die um den rohrförmigen Körper herum in einer zu dessen Achse orthogonalen Ebene angeordnet ist, in ihrer Mitte mit Desinfektionsflüssigkeit gespeist ist und in deren beiden Enden zwei axiale Leitungen münden, deren jeweils anderes Ende in die Mitte einer kreisförmigen, sich über 90° in einer zur Achse des rohrförmigen Körpers transversalen Ebene erstreckenden Leitung (17) mündet, wobei die beiden Enden jeder dieser letzteren Leitungen mit einem Injektor (6) in Verbindung stehen oder mit einer Leitung, deren Enden jeweils einen Injektor speisen, gemäß der Anzahl der Injektoren, die die Vorrichtung umfaßt.

2. Vorrichtung nach Anspruch 1, dadurch gekennzeichnet, daß die Desinfektionsflüssigkeits-Zufuhrmittel eine erste kreisförmige, sich über 180° erstreckende Leitung umfassen, die um den rohrförmigen Körper herum in einer zu dessen Achse orthogonalen Ebene angeordnet ist, in ihrer Mitte mit Desinfektionsflüssigkeit gespeist ist und in deren beiden Enden zwei axiale Leitungen münden, deren jeweils anderes Ende in die Mitte einer kreisförmigen, sich über 90° in einer zur Achse des rohrförmigen Körpers transversalen Ebene erstreckenden Leitung mündet, wobei die beiden Enden jeder dieser letzteren Leitungen mit einem Injektor in Verbindung stehen.

3. Vorrichtung nach Anspruch 2, dadurch gekennzeichnet, daß die Luft- und Desinfektionsflüssigkeits-Zufuhrmittel von drei nebeneinander angeordneten und paarweise miteinander verbundenen Kränzen (7, 8, 9) gebildet sind, in deren einander zugewandten Flächen Nuten vorgesehen sind, welche dazu bestimmt sind, die Bildung ringförmiger Kanäle sicherzustellen, wobei der erste Kranz (7) zwei axiale Durchgänge zur Verbindung mit mit Flüssigkeit und Luft versorgten Leitungen umfaßt, wobei die ersten und zweiten Kränze (7, 8) einen ersten sich über einen Winkel von 180° erstreckenden Kanal (15) begrenzen, um den Durchtritt von Flüssigkeit zu erlauben, wobei der zweite Kranz (8) drei axiale Durchgänge umfaßt, einen (13) für den direkten Durchtritt von Luft und zwei (16) für den Durchtritt von Flüssigkeit, wobei die zweiten und dritten Kränze (8, 9) einenteils auf 306° den kreisförmigen Kanal (14) zur Verteilung von Luft zu den Injektoren (6) und andernteils auf 90° jeweils zwei Kanäle (17) zur Verteilung von Flüssigkeit begrenzen.

4. Vorrichtung nach Anspruch 3, dadurch gekennzeichnet, daß die drei Kränze (7, 8, 9) aus Kunststoff gefertigt sind und ihre einander zugewandten Flächen Zusammenfügemittel umfassen, welche von einer Mehrzahl einander gegenüberliegender kreisförmiger Wülste (19) gebildet sind, von denen jede von zwei Vertiefungen (20) eingefaßt ist, wobei jede zur Bildung eines Kanals bestimmte Nut zwischen zwei benachbarten Wülsten verschiedenen Durchmessers angeordnet ist.

## Claims

1. A device for cleaning and disinfecting medical and surgical instruments, of the type including a tubular body (2) of which one end is open and the other end opens into a reservoir for collecting used liquids, and of which the internal wall is provided with nozzles (5) for diffusing a nebulised disinfecting product, each nozzle being associated with an injector (6) to which are supplied the disinfecting liquid and air under pressure, characterised in that the means for supplying air to the injectors (6) comprises a circular annular conduit (14) extending over 360° disposed around the tubular body in a plane transverse to the axis of the latter, supplying directly the different injectors (6), and in that the means for supplying disinfecting liquid comprises a first circular conduit (15) extending over 180° disposed around the tubular body in a plane perpendicular to the axis of the latter, supplied in its central region by the disinfecting liquid, and into the two ends of which open two axial conduits, the other end of each of which opens into the central region of a circular conduit (17) extending over 90° in a plane transverse to the axis of the tubular body, the two ends of each of these latter conduits communicating with an injector (6), or with a conduit of which the ends each supply an injector, according to the number of injectors included in the device.

2. A device according to Claim 1, characterised in that the means for supplying disinfecting liquid comprise a first circular conduit extending over 180° disposed around the tubular body in a plane perpendicular to the axis of the latter, supplied in its central region by the disinfecting liquid, and into the two ends of which open two axial conduits, the other end of each of which opens into the central region of a circular conduit extending over 90° in a plane transverse to the axis of the tubular body, the two ends of each of these latter conduits communicating with an injector.

3. A device according to Claim 2, characterised in that the means for supplying air and disinfecting liquid are constituted by three rings (7, 8, 9) which are juxtaposed and connected in pairs, in the opposing faces of which are formed grooves intended for the formation of circular channels, the first ring (7) comprising two axial through holes for connection to liquid and air supply conduits, the first and second rings (7, 8) delimiting a first channel (15) extending over an angle of 180°, to permit the passage of liquid, the second ring (8) including three axial through holes of which one (13) serves for the direct passage of air and two (16) for the passage of liquid, the second and third rings (8, 9) on the one hand, delimiting over 360°, the circular air distribution channel (14) for the injectors (6), and on the other hand, over 90° each delimiting two liquid distribution channels (17).

4. A device according to Claim 3, characterised in that the three rings (7, 8, 9) are made from synthetic material and their opposing faces include assembly means constituted by a plurality of circular opposing flanges (19), each of which is bordered by two recesses (20), each groove intended for the formation of a channel being disposed between two adjacent flanges and of different diameter.
